Europäisches Patentamt

(19) European Patent Office

Office européen des brevets

(11) Veröffentlichungsnummer: **0 374 708**

**A1**

## (12) EUROPÄISCHE PATENTANMELDUNG

(21) Anmeldenummer: **89123006.2**

(22) Anmeldetag: **13.12.89**

(51) Int. Cl.⁵: **C12M 1/00, C02F 11/04**

(30) Priorität: **13.12.88 DE 3841885**

(43) Veröffentlichungstag der Anmeldung:
**27.06.90 Patentblatt 90/26**

(84) Benannte Vertragsstaaten:
**AT BE CH DE ES FR GB GR IT LI LU NL SE**

(71) Anmelder: **Probst, Josef**
**Köckstrasse 9**
**D-8360 Deggendorf(DE)**

(72) Erfinder: **Probst, Josef**
**Köckstrasse 9**
**D-8360 Deggendorf(DE)**

(74) Vertreter: **Gustorf, Gerhard, Dipl.-Ing.**
**Patentanwalt Dipl.-Ing. Gerhard Gustorf**
**Bachstrasse 6 A**
**D-8300 Landshut(DE)**

(54) **Biogasreaktor.**

(57) Vorrichtung zur Erzeugung von Biogas aus organischem Substrat mit einer dieses aufnehmenden, zylindrischen und liegend angeordneten Reaktortrommel (30), die eine Förderschnecke (32) enthält, deren Drehachse (34) in der Längsachse der Reaktortrommel (30) liegt. Ferner sind ein Einfüllbehälter (48) für das frische Substrat, der über ein Zuführrohr (64) mit der Reaktortrommel (30) verbunden ist, ein Abgaberohr (70) für das ausgefaulte Substrat, eine Entnahmeeinrichtung für das erzeugte Biogas sowie eine Heizeinrichtung (82) zur Erzeugung der Prozeßwärme vorgesehen. Die Reaktortrommel (30), der Einfüllbehälter (48) und die Heizeinrichtung (82) sind in einem gemeinsamen Gehäuse (10) untergebracht, dessen Wände (12, 14, 16, 18) und Deckel (20, 22) wärmedämmend ausgebildet sind und das teilweise mit einer Flüssigkeit (80) zur Abgabe der Prozeßwärme an die Reaktortrommel (30) gefüllt ist.

Fig. 1

EP 0 374 708 A1

## Biogasreaktor

Die Erfindung betrifft eine Vorrichtung zur Erzeugung von Biogas aus organischem Substrat mit einer dieses aufnehmenden, zylindrischen und liegend angeordneten Reaktortrommel, die eine Förderschnecke enthält, deren Drehachse in der Längsachse der Reaktortrommel liegt, mit einem Einfüllbehälter für das frische Substrat, der über ein Zuführrohr mit der Reaktortrommel verbunden ist, mit einer Abführeinrichtung für das ausgefaulte Substrat, mit einer Entnahmeeinrichtung für das erzeugte Biogas sowie mit einer Heizeinrichtung zur Erzeugung der Prozeßwärme.

Ein derartiger Biogasreaktor ist aus der EP-A 113 719 bekannt. Die bekannte Vorrichtung eignet sich hervorragend für die Erzeugung von Biogas in Großbetrieben o. dgl., weshalb sie so konzipiert ist, daß sie fest installiert ist. Die Reaktortrommel ist dabei über Verbindungsleitungen mit einem Vorsatzbehälter verbunden, der sowohl den Einfüllbehälter für das frische Substrat als auch die Heizeinrichtung aufweist. Die Heizeinrichtung ist ihrerseits mit der Trommelwandung verbunden. Wesentlich ist dabei, daß das Zuführrohr für das frische Substrat vom Einfüllbehälter durch die gesamte Reaktortrommel hindurch verläuft, so daß sein Auslauf an der gegenüberliegenden Stirnwand in die Trommel mündet.

Der Erfindung liegt die Aufgabe zugrunde, einen Biogasreaktor zur Verfügung zu stellen, der aufgrund einer kompakten Bauweise besonders für den Einsatz in kleinen oder mittleren Betrieben geeignet ist.

Gemäß der Erfindung wird diese Aufgabe bei einer Vorrichtung der eingangs umrissenen Gattung dadurch gelöst, daß die Reaktortrommel, der Einfüllbehälter und die Heizeinrichtung in einem gemeinsamen Gehäuse untergebracht sind, dessen Wände und Deckel wärmedämmend ausgebildet sind und das teilweise mit einer Flüssigkeit zur Abgabe der Prozeßwärme an die Reaktortrommel gefüllt ist.

Eine derartige Vorrichtung läßt sich als eine kompakte Baueinheit herstellen, die bei Bedarf an beliebigen Orten aufgestellt werden kann, um durch die Entsorgung von anfallendem Substrat in umweltfreundlicher Weise Energie zu erzeugen, beispielsweise zu Heizzwecken, zur Warmwasserbereitung oder zur Stromerzeugung. Unter den zahlreichen Einsatzmöglichkeiten seien kleine und mittlere Bauernhöfe, Freizeitanlagen, Gastronomieeinrichtungen oder Hausgemeinschaften herausgegriffen.

Sinnvolle Baugrößen für die Reaktortrommel liegen zwischen 3 und 40 m³.

In vorteilhafter Weiterbildung der Erfindung bestehen Wände und Deckel des Gehäuses in Sandwichbauweise aus einer Innenschicht und einer Außenschicht aus Kunststoff sowie aus einer Zwischenschicht aus Schaumkunststoff.

Es ist besonders günstig, wenn der Deckel im Bereich der Reaktortrommel an seiner Innenseite einen Speicherraum für das erzeugte Biogas enthält.

Nach einem weiteren Merkmal der Erfindung weist der Einfüllbehälter einen Trichterboden auf, der mit einer Substratpumpe verbunden ist, deren Abgabestutzen in das Zuführrohr führt, welches in die dem Einfüllbehälter zugewandte Stirnwand der Reaktortrommel mündet. Es ergibt sich dabei ein fließender Reaktionsablauf mit kontinuierlichem Übergang von einer Reaktionsphase zur nächsten Reaktionsphase des anaeroben Fermentationsprozesses im Bereich der Förderschnecke. Da die Kammern der Förderschnecke zwischen den einzelnen Windungen in sich als abgeschlossen zu betrachten sind, wird ein biochemischer Mehrphasenbetrieb mit fließendem Übergang von der essigsauren Phase zur Methanphase am Ende der Reaktortrommel erzielt.

Für die störungsfreie Zuführung des eingefüllten Substrates ist es günstig, wenn im Einfüllbehälter ein Schneid- und Rührwerk für das Substrat angeordnet ist.

Eine besonders vorteilhafte Weiterbildung der Erfindung besteht darin, das unterhalb der Reaktortrommel ein Absaugrohr für das Substrat angebracht ist, das über auf die Trommellänge verteilte Entnahmeventile mit dem Innenraum der Reaktortrommel verbunden ist und das über eine Mischpumpe zu einem Zumischrohr führt, welches über Zumischventile mit der Reaktortrommel verbunden ist. Auf diese Weise kann an nahezu jeder gewünschten Stelle der Reaktortrommel Substrat entnommen und der Trommel an einer anderen Stelle wieder zugeführt werden, um durch einen solchen Impfvorgang die Säurephase zu regeln und den anaeroben Fermentationsprozeß zu optimieren.

Nach einem anderen Merkmal der Erfindung sind an der Zylinderwand der Reaktortrommel zwei spiralartig verlaufende Rohre für die Flüssigkeit zur Abgabe der Prozeßwärme angebracht, die für den Gegenstrombetrieb mit in Längsrichtung verlaufenden Vorlauf- und Rücklaufleitungen verbunden sind. Mit dieser Maßnahme wird eine gegenläufige Doppelheizung erzielt, die eine optimale Temperaturverteilung für den Reaktionsprozeß ermöglicht.

Weitere Merkmale und Vorteile der Erfindung ergeben sich aus den Patentansprüchen und aus der nachfolgenden Beschreibung von Ausführungsbeispielen, die in der Zeichnung dargestellt sind.

Es zeigen:

Figur 1 die teilweise aufgeschnittene Ansicht eines Biogasreaktors nach der Erfindung,

Figur 2 die Stirnansicht des Reaktors der Figur 1,

Figur 3 einen Längsschnitt durch den Biogasreaktor,

Figur 4 einen Querschnitt in der Ebene IV - IV der Figur 3,

Figur 5 einen Querschnitt in der Ebene V - V der Figur 3,

Figur 6 einen Querschnitt in der Ebene VI - VI der Figur 3,

Figur 7 einen Längsschnitt durch den Deckel des Biogasreaktors der Figuren 1 bis 6,

Figur 8 einen Längsschnitt durch eine abgeänderte Ausführungsform der Reaktortrommel,

Figur 9 die perspektivische und teilweise aufgeschnittene Ansicht einer weiteren Ausführungsform mit zwei Reaktortrommeln und

Figur 10 die schematische Teildarstellung einer Reaktortrommel der Figur 9.

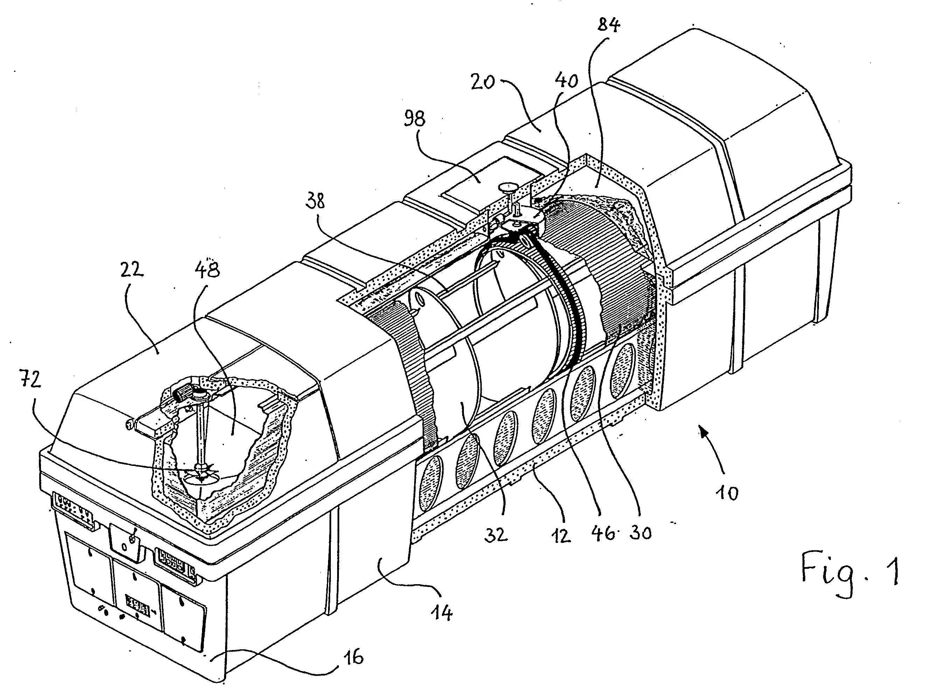

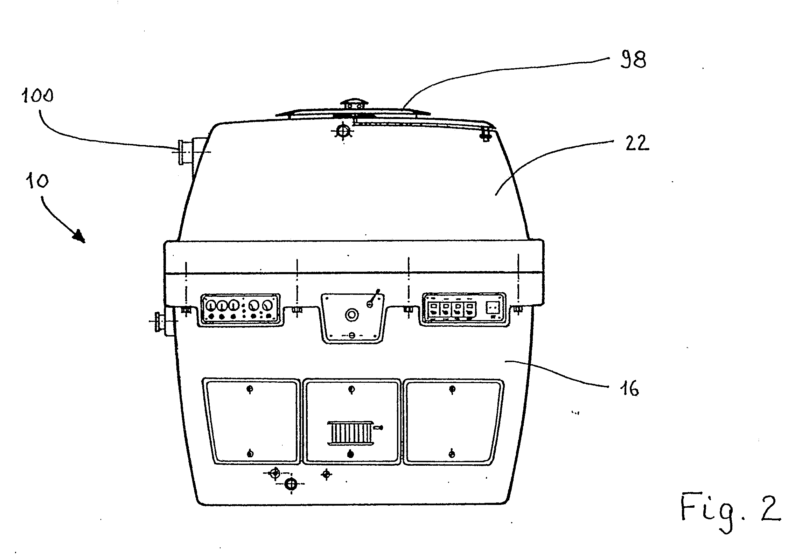

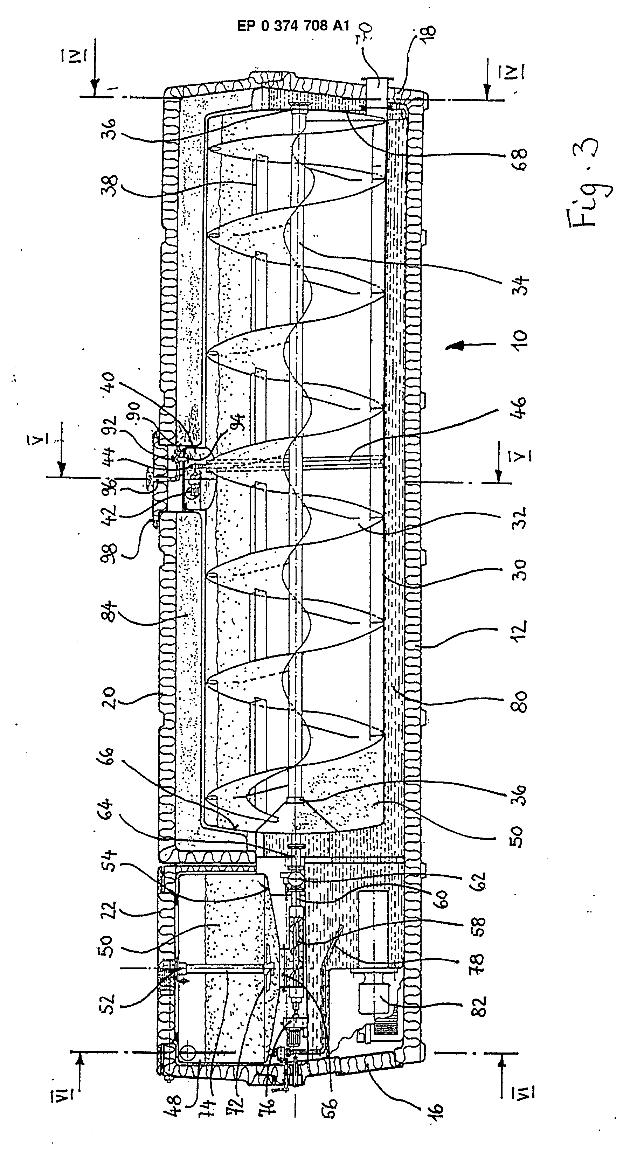

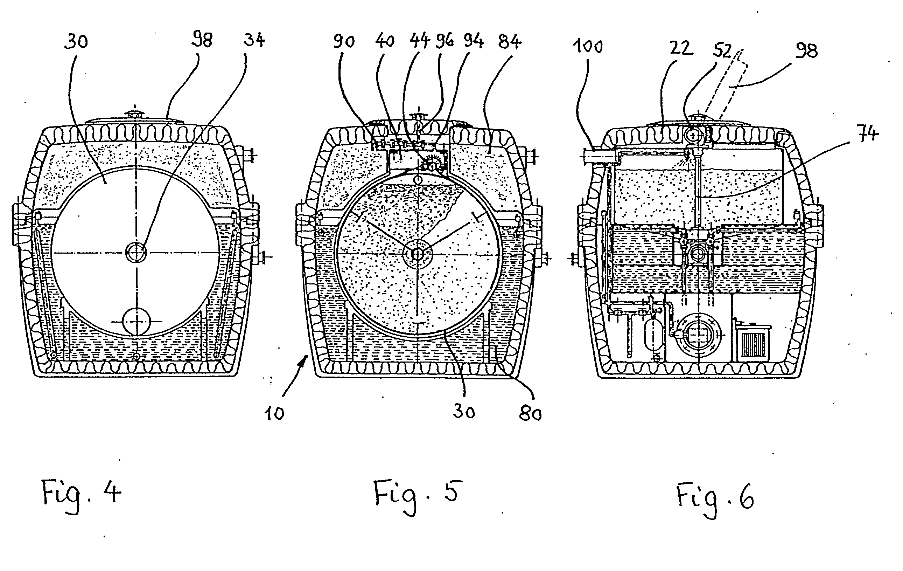

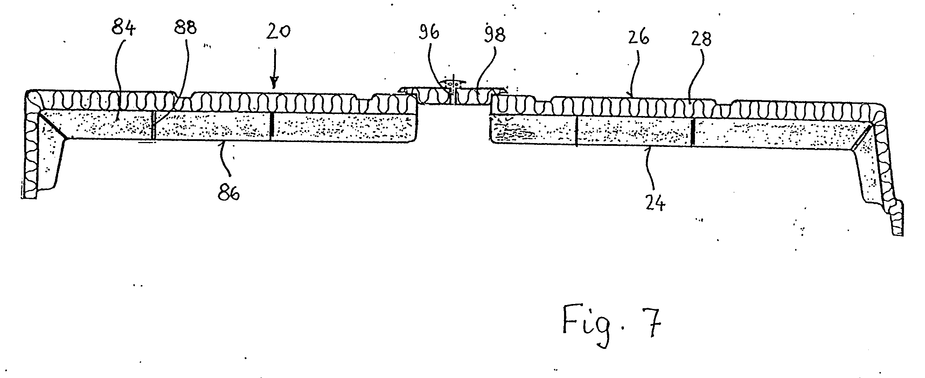

Die in den Figuren 1 bis 7 gezeigte Vorrichtung gemäß der Erfindung dient zur Erzeugung von Biogas aus organischem Substrat. Sie hat ein wannenförmiges Gehäuse 10, dessen Boden 12, Seitenwände 14 und Stirnwände 16 und 18 in Sandwichbauweise hergestellt sind; diese sowie zwei Deckel 20 und 22 bestehen, wie im Beispiel des Deckels 20 der Figur 7 gezeigt ist, aus einer Innenschicht 24, einer Außenschicht 26 und einer Zwischenschicht 28. Die Zwischenschicht 28 wird durch einen wärmeisolierenden Schaumkunststoff gebildet, vorzugsweise PU-Schaum. Die Innenschicht 24 ist ein gasdichter Kunststoff, während die Außenschicht 26 aus einem UV-beständigen Polyester besteht.

Der in Figur 7 gezeigte Deckel 20 nimmt den größten Teil der Länge des Gehäuses 10 ein. Auf diesem Teil der Länge ist im Gehäuse 10 eine Reaktortrommel 30 fest angebracht, die zylindrisch ausgebildet ist und deren Längsachse sich waagrecht erstreckt. In der Reaktortrommel 30 ist eine Förderschnecke 32 drehbar gelagert, deren Drehachse 34 in der Längsachse der Reaktortrommel 30 liegt. An beiden Seiten ist die Drehachse 34 in Lagern 36 gelagert.

Als Abstandselemente zwischen den einzelnen Windungen der Förderschnecke 32 dienen winkelförmige Leisten 38, die die Förderschnecke 32 stabilisieren und gleichzeitig den Rühr- und Durchmischungseffekt der Förderschnecke 32 erhöhen.

Im mittleren Bereich hat die Reaktortrommel 30 an ihrer Oberseite einen Gasdom 40, der nach oben abgeschlossen ist und einen Motor 42 mit Getriebe 44 aufnimmt. Der Motor 42 treibt über das Getriebe 44 und einen Riemen- oder Kettenantrieb 46, der am Außenumfang der Förderschnecke 32 angreift, die Förderschnecke an. Die Drehzahl des Motors 42 kann den jeweiligen Arbeitsbedingungen der Vorrichtung angepaßt werden.

Der in Figur 3 linke Teil des Gehäuses 10 nimmt in der oberen Hälfte einen Einfüllbehälter 48 für das frische Substrat 50 auf und ist durch den Deckel 32 abgeschlossen. Der Einfüllbehälter 48 hat einen Trichterboden 54, dessen mittlerer Bereich mit einem Zuführstutzen 56 einer Substratpumpe 58 verbunden ist. Der Abgabestutzen 60 der Substratpumpe 58 ist über ein Dreiwegeventil 62 mit einem Zuführrohr 64 verbunden, das in die dem Einfüllbehälter 48 zugewandte Stirnwand 66 der Reaktortrommel 30 mündet. An der gegenüberliegenden Stirnwand 68 der Reaktortrommel 30 ist im unteren Bereich ein Abgaberohr 70 für das ausgefaulte Substrat angebracht, das durch die Stirnwand 18 des Gehäuses 10 nach außen führt.

Die Figuren 1 und 3 zeigen weiter, daß im Einfüllbehälter 48 ein Schneid- und Rührwerk 72 mit senkrechter Welle 74 untergebracht ist, deren Drehantrieb 52 im Deckel 52 angeordnet ist. Das Schneid- und Rührwerk 72 befindet sich dicht über dem Zuführstutzen 56 und sorgt für eine gute Zerkleinerung und Durchmischung des zugeführten Substrates 50, bevor dieses in die Substratpumpe 58 gelangt.

Unterhalb des Einfüllbehälters 48 ist eine Umwälzpumpe 76 befestigt, deren Saugseite im Bereich der vom Einfüllbehälter 48 entfernten Stirnwand 68 der Reaktortrommel 30 liegt, während die Druckseite in Form eines Rohres 78 unter der Substratpumpe 58 liegt. Wie Figur 3 zeigt, ist das Gehäuse 10 bis über die Mittelebene mit Wasser 80 gefüllt, das von einer Heizeinrichtung 82 erwärmt wird. Die auf diese Weise erzeugte Prozeßwärme wird mittels der Umwälzpumpe 76 durch das Wasser 80 gleichmäßig auf die Reaktortrommel 30 übertragen. Die Heizeinrichtung 82 kann als Wärmetauscher, Brenner oder integrierter Gasmotor ausgebildet sein. Die Umwälzpumpe 76 saugt dabei das Wasser vom im Bereich der Stirnwand 68 liegenden, hinteren Temperaturtotpunkt ab und sorgt für eine möglichst gleichmäßige, konstante Temperatur.

Die Figuren 3 und 7 zeigen schließlich, daß der Deckel 20 an seiner Innenseite einen Speicherraum 84 für das erzeugte Biogas enthält. Dieser Speicherraum 84 hat eine nach innen weisende Wand 86 aus Kunststoff und/oder Stahl, die durch Versteifungsstreben 88 verstärkt ist, so daß Druckausbeulungen vorgebeugt wird.

Der Speicherraum 84 ist über eine Leitung 90 mit einem Dreiwegeventil 92 verbunden, von dem ein Anschluß 94 in den Gasdom 40 führt, während ein weiterer Anschluß 96 zur Entnahme des erzeugten Biogases dient. Mit diesen Maßnahmen kann das erzeugte Biogas entweder im Speicherraum 84 zwischengespeichert oder direkt über den Entnahmeanschluß 96, der durch einen Isolierdeckel 98 innerhalb des Deckels 20 führt, abgenommen werden.

Das frische Substrat 50, das bei geöffnetem Deckel 52 in den Einfüllbehälter 48 zugeführt worden ist, wird über die Substratpumpe 58 und das Zuführrohr 64 in die Reaktortrommel 30 gefördert. Die Förderschnecke 32 bewegt das Substrat aus der Anfangsphase (essigsauer) bis zu der Stirnwand 68 (Methanphase), wo das Substrat durch das Abgaberohr 70 abgezogen werden kann. Die Temperatur des Wassers 80 im Gehäuse 10 wird auf einem gewünschten Wert gehalten, der im allgemeinen zwischen 28° und 60° C liegt.

Ein seitlicher Stutzen 100 im Deckel 22 dient für die Zufuhr von dünnflüssigen Gärmedien oder Abwasser.

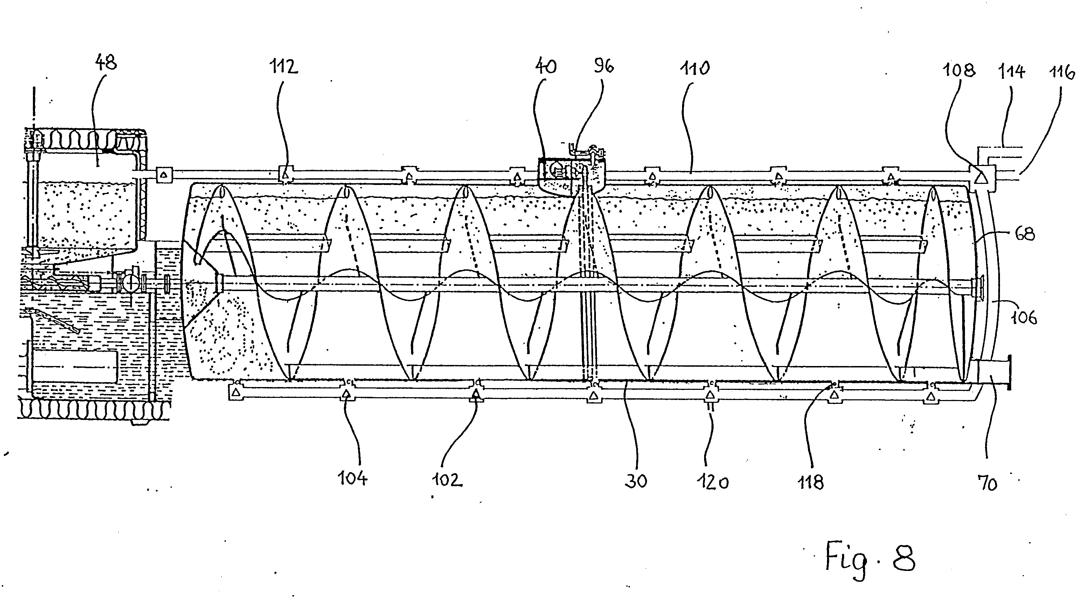

Bei der in Figur 8 gezeigten Variante der Erfindung ist unterhalb der Reaktortrommel 30 ein Absaugrohr 102 für das Substrat angebracht, welches sich in Längsrichtung der Reaktortrommel 30 erstreckt. Gleichmäßig auf die Trommellänge ist das Absaugrohr 102 über Entnahmeventile 104 mit dem Innenraum der Reaktortrommel 30 verbunden. Im Bereich der Stirnwand 68 der Reaktortrommel 30 ist das Absaugrohr 102 über ein Verbindungsrohr 106 mit einer Mischpumpe 108 verbunden, die zu einem Zumischrohr 110 führt. Das Zumischrohr 110 verläuft etwa parallel zu dem Absaugrohr 102 oberhalb der Reaktortrommel 30 und ist über Zumischventile 112, die ebenfalls etwa gleichmäßig über die Reaktorlänge verteilt sind, mit dem Innenraum der Reaktortrommel 30 verbunden. An die Mischpumpe 108 sind eine Wasserleitung 114 sowie eine Zumischleitung 116 für Bioprozeßhilfsmittel angeschlossen.

Im Bereich der Entnahmeventile 104 sind Sensoren 118 angebracht, die in das Innere der Reaktortrommel 30 eingreifen und zum Messen von Prozeßdaten (Temperatur, Druck, Säuregrad o. dgl. dienen. Über diese Sensoren 118 können sowohl die Entnahmeventile 104 als auch die Zumischventile 112 gesteuert werden, um dadurch an einer bestimmten Stelle der Reaktortrommel 30 Substrat zu entnehmen und über ein bestimmtes Zumischventil 112 wieder einzuimpfen. Über die Sensoren 118 und die Mischpumpe 108 kann ferner die Zufuhr von Wasser oder Bioprozeßhilfsmitteln durch die Wasserleitung 114 bzw. die Zumischleitung 116 gesteuert werden.

In Figur 8 ist angedeutet, daß die Entnahmeventile 104 Stutzen 120 zur Probenentnahme aufweisen können.

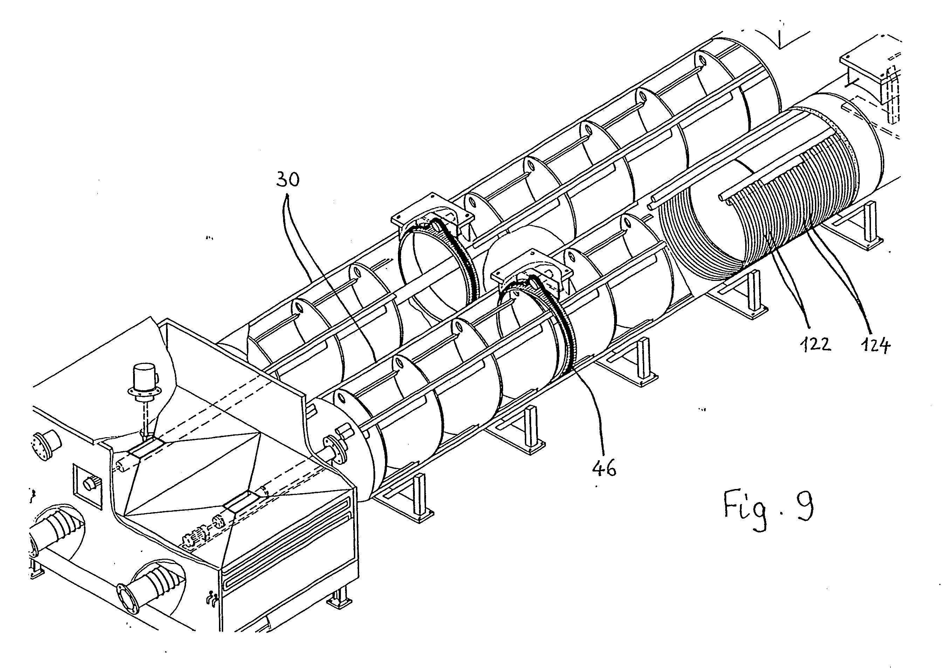

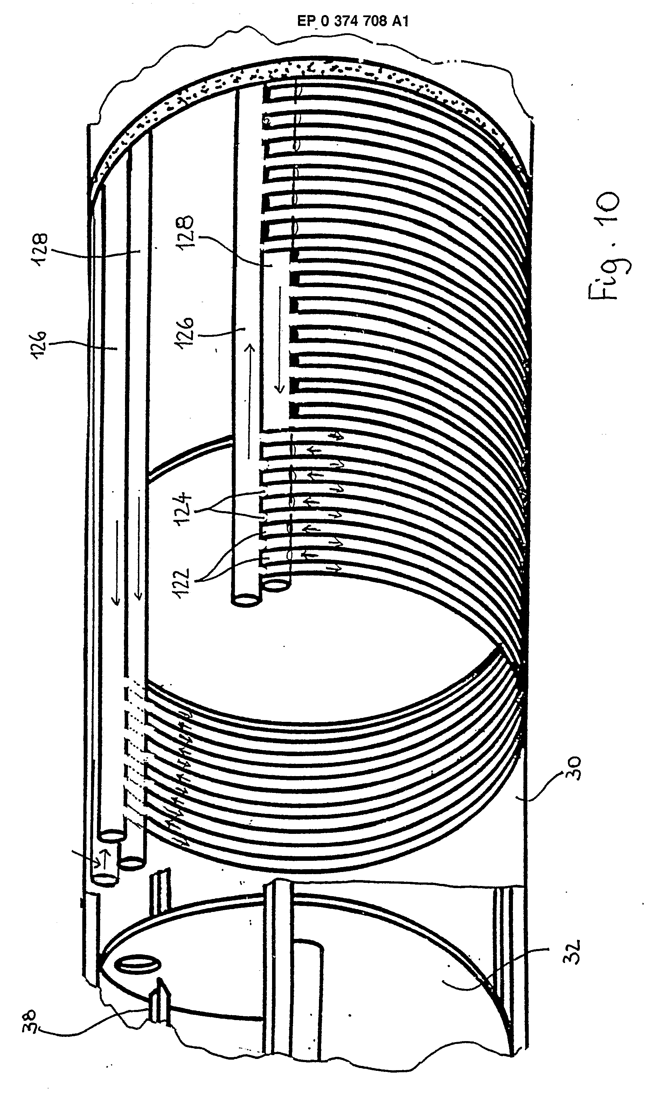

Die Figuren 9 und 10 zeigen schließlich eine Ausführungsmöglichkeit mit zwei parallel nebeneinander angebrachten Reaktortrommeln 30, an deren Zylinderwand zwei spiralartig verlaufende Rohre

122, 124 für die Flüssigkeit zur Abgabe der Prozeßwärme angebracht sind. Die beiden Rohrsysteme 122, 124 sind für den Gegenstrombetrieb mit jeweils einer in Längsrichtung verlaufenden Vorlaufleitung 126 und einer Rücklaufleitung 128 verbunden.

Mit Hilfe der erfindungsgemäß ausgebildeten Vorrichtung erfolgt trotz einer Trennung zwischen frischem Substrat und Ausgangssubstrat eine gute Durchmischung von Sink- und Schwimmschichten, wobei der Ablauf von der einen zur nächsten Reaktionsphase fließend erfolgt. Die Durchlaufzeit des Substrates durch die Reaktortrommel 30 kann den Erfordernissen und Prozeßbedingungen entsprechend gesteuert werden, wobei auch eine zusätzliche Impfung des Substrates möglich ist.

## Ansprüche

1. Vorrichtung zur Erzeugung von Biogas aus organischem Substrat mit einer dieses aufnehmenden, zylindrischen und liegend angeordneten Reaktortrommel, die eine Förderschnecke enthält, deren Drehachse in der Längsachse der Reaktortrommel liegt, mit einem Einfüllbehälter für das frische Substrat, der über ein Zuführrohr mit der Reaktortrommel verbunden ist, mit einer Abführeinrichtung für das ausgefaulte Substrat, mit einer Entnahmeeinrichtung für das erzeugte Biogas sowie mit einer Heizeinrichtung zur Erzeugung der Prozeßwärme, dadurch **gekennzeichnet,** daß die Reaktortrommel (30), der Einfüllbehälter (48) und die Heizeinrichtung (82) in einem gemeinsamen Gehäuse (10) untergebracht sind, dessen Wände (12, 14, 16, 18) und Deckel (20, 22) wärmedämmend ausgebildet sind und das teilweise mit einer Flüssigkeit (80) zur Abgabe der Prozeßwärme an die Reaktortrommel (30) gefüllt ist.

2. Vorrichtung nach Anspruch 1, dadurch gekennzeichnet, daß Wände (14, 16, 18) und Deckel (20, 22) des Gehäuses (10) in Sandwichbauweise aus einer Innenschicht (24) und einer Außenschicht (26) aus Kunststoff sowie einer Zwischenschicht (28) aus Schaumkunststoff bestehen.

3. Vorrichtung nach Anspruch 1 oder 2, dadurch gekennzeichnet, daß der Deckel (20) im Bereich der Reaktortrommel (30) an seiner Innenseite einer Speicherraum (84) für das erzeugte Biogas enthält.

4. Vorrichtung nach Anspruch 3, dadurch gekennzeichnet, daß der Speicherraum (84) über ein Dreiwegeventil (92) mit einem Gasdom (40) der Reaktortrommel (30) verbunden ist.

5. Vorrichtung nach Anspruch 4, dadurch gekennzeichnet, daß innerhalb des Gasdomes (40) ein Motor (42) für den Drehantrieb der Förderschnecke (32) untergebracht ist.

6. Vorrichtung nach einem der vorhergehenden Ansprüche, dadurch gekennzeichnet, daß der Einfüllbehälter (48) einen Trichterboden (54) aufweist, der mit einer Substratpumpe (58) verbunden ist, deren Abgabestutzen (60) in das Zuführrohr (64) führt, welches in die dem Einfüllbehälter (48) zugewandte Stirnwand (66) der Reaktortrommel (30) mündet.

7. Vorrichtung nach Anspruch 6, dadurch gekennzeichnet, daß an der vom Einfüllbehälter (48) entfernten Stirnwand (68) der Reaktortrommel (30) ein Abgaberohr (70) für das ausgefaulte Substrat angebracht ist.

8. Vorrichtung nach einem der vorhergehenden Ansprüche, dadurch gekennzeichnet, daß im Einfüllbehälter (48) ein Schneid- und Rührwerk (72) für das Substrat angeordnet ist.

9. Vorrichtung nach einem der vorhergehenden Ansprüche, dadurch gekennzeichnet, daß unter dem Einfüllbehälter (48) eine Umwälzpumpe (76) für die Flüssigkeit untergebracht ist, deren Saugseite im Bereich der vom Einfüllbehälter (48) entfernten Stirnwand (68) der Reaktortrommel (30) liegt.

10. Vorrichtung nach einem der vorhergehenden Ansprüche, dadurch gekennzeichnet, daß unter dem Einfüllbehälter (48) die Heizeinrichtung (82) untergebracht ist.

11. Vorrichtung nach einem der vorhergehenden Ansprüche, dadurch gekennzeichnet, daß unterhalb der Reaktortrommel (30) ein Absaugrohr (102) für das Substrat angebracht ist, das über auf die Trommellänge verteilte Entnahmeventile (104) mit dem Innenraum der Reaktortrommel (30) verbunden ist und das über eine Mischpumpe (108) zu einem Zumischrohr (110) führt, welches über Zumischventile (112) mit der Reaktortrommel (30) verbunden ist.

12. Vorrichtung nach Anspruch 11, dadurch gekennzeichnet, daß die Entnahmeventile (108) und die Zumischventile (112) durch Meßeinrichtungen steuerbar sind.

13. Vorrichtung nach Anspruch 12, dadurch gekennzeichnet, daß die Meßeinrichtungen Sensoren (118) aufweisen, die im Bereich der Entnahmeventile (104) in die Reaktortrommel (30) ragen.

14. Vorrichtung nach einem der Ansprüche 11 bis 13, dadurch gekennzeichnet, daß die Entnahmeventile (104) Stutzen (120) zur Probenentnahme aufweisen.

15. Vorrichtung nach einem der vorhergehenden Ansprüche, dadurch gekennnzeichnet, daß an der Zylinderwand der Reaktortrommel (30) zwei spiralartig verlaufende Rohre (122, 124) für die Flüssigkeit zur Abgabe der Prozeßwärme angebracht sind, die für den Gegenstrombetrieb mit in Längsrichtung verlaufenden Vorlauf- und Rücklaufleitungen (126, 128) verbunden sind.

Fig. 1

Fig. 2

EP 0 374 708 A1

Fig. 3

Fig. 6

Fig. 5

Fig. 4

Fig. 7

EP 0 374 708 A1

48  112  40  96  110  108  114  116

68

106

104  102  30  120  118  70

EP 0 374 708 A1

Fig. 8

30

122 124

46

Fig. 9

EP 0 374 708 A1

Fig. 10

EP 0 374 708 A1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | FR-A-2 485 035 (MAUMONT et al.) <br> * Das ganze Dokument * <br> --- | 1-15 | C 12 M 1/00 <br> C 02 F 11/04 |
| A | FR-A-2 480 305 (CAMPOLO) <br> * Das ganze Dokument * <br> --- | 1-15 | |
| A | WO-A-8 605 171 (SZIKRISZT) <br> * Ansprüche 1-13; Figur 1 * <br> ----- | 1-15 | |

RECHERCHIERTE SACHGEBIETE (Int. Cl.5)

C 12 M
C 02 F

Der vorliegende Recherchenbericht wurde für alle Patentansprüche erstellt

| Recherchenort | Abschlußdatum der Recherche | Prüfer |
|---|---|---|
| DEN HAAG | 21-03-1990 | EPAILLARD P.J.H.M. |

KATEGORIE DER GENANNTEN DOKUMENTE

X : von besonderer Bedeutung allein betrachtet
Y : von besonderer Bedeutung in Verbindung mit einer
　　anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
　　nach dem Anmeldedatum veröffentlicht worden ist
D : in der Anmeldung angeführtes Dokument
L : aus andern Gründen angeführtes Dokument
...........................................................................
& : Mitglied der gleichen Patentfamilie, übereinstimmendes
　　Dokument